# EUROPEAN PATENT SPECIFICATION

(11) **EP 4 188 377 B1**
(45) Date of publication and mention of the grant of the patent: **17.01.2024**
(21) Application number: 22718776.2
(22) Date of filing: 23.03.2022
(51) Int. Cl.: A61K 31/4706, A61K 31/155, A61K 31/366, A61K 31/40, A61K 45/06, A61P 35/02

(54) **COMBINATION COMPRISING CHLOROQUINE, METFORMIN AND STATIN FOR MANAGEMENT OF CANCER, COMPOSITION AND METHODS THEREOF**
KOMBINATION MIT CHLOROQUIN, METFORMIN UND STATIN ZUR BEHANDLUNG VON KREBS, ZUSAMMENSETZUNG UND VERFAHREN DAFÜR
COMBINAISON COMPRENANT DE LA CHLOROQUINE, DE LA METFORMINE ET DE LA STATINE POUR LE TRAITEMENT DU CANCER, COMPOSITION ET MÉTHODES ASSOCIÉES

(30) Priority: 23.03.2021 US 202163164828 P
(43) Date of publication of application: 07.06.2023
(73) Proprietor: Cellworks Group, Inc., San Jose, CA 95110 (US)
(72) Inventor: KUMAR, Ansu, Bangalore, Karnataka, 560066 (IN); KHANDELWAL, Swati, Bangalore, Karnataka, 560066 (IN); MOHAPATRA, Subrat, Bangalore, Karnataka, 560067 (IN); GROVER, Himanshu, Delhi, 110085 (IN); PATIL, Vivek, Belgaum, Karnataka, 590017 (IN); TYAGI, Anuj, Bangalore, Karnataka, 560067 (IN); KUMAR AGRAWAL, Ashish, Bangalore, Karnataka, 560036 (IN); KAPOOR, Shweta, Bangalore, Karnataka, 560066 (IN); MUNDKUR, Yatin, Los Altos Hills, CA 94022 (US)
(74) Representative: CMS Cameron McKenna Nabarro Olswang LLP
(86) International application number: PCT/IB2022/052662
(87) International publication number: WO 2022/201065

(56) References cited:
- WO-A2-2015/068142
- EA-B1- 026 317
- KR-A- 20060 092 428
- US-A1- 2018 169 242
- US-B2- 10 413 558
- PRITHVIRAJ BOSE ET AL: "Investigational histone deacetylase inhibitors (HDACi) in myeloproliferative neoplasms", EXPERT OPINION ON INVESTIGATIONAL DRUGS, vol. 25, no. 12, 31 October 2016 (2016-10-31), pages 1393-1403, XP055565134, UK ISSN: 1354-3784, DOI: 10.1080/13543784.2016.1250882

## Description

### TECHNICAL FIELD

The present disclosure relates to the field of oncology. In particular, the disclosure relates to a combination therapy comprising chloroquine, metformin and a statin or respective pharmaceutically acceptable salts or derivatives thereof, for use in the management of Myeloproliferative neoplasms (MPNs) or any associated condition. The present disclosure also relates to a composition comprising said combination, and a method for use in the management of MPN or any associated condition by employing said composition or combination therapy. The present disclosure also provides a combination of compounds for use in preparation of a medicament for managing MPN or any associated condition.

### BACKGROUND OF THE DISCLOSURE

Myeloproliferative neoplasms (MPNs) are a subset of myeloid malignancies that are characterized by the expansion of a multipotent hematopoietic progenitor stem cell. The World Health Organization classifies chronic myeloid leukemia (CML), polycythemia vera (PV), essential thrombocythemia (ET), and primary myelofibrosis (PMF) as classic MPNs. The most prominent mutations in MPNs are in the genes that encode for JAK2, MPL and CALR. Of these, the JAK2-V617F mutation was the first identified, and is present in about 95% of PV, and about 55% and 60% of ET and PMF patients, respectively. MPL mutations are present in about 3% ET patients and about 7% of PMF patients. Interestingly, CALR mutations are present in the majority of ET and PMF patients who lack JAK2 and MPL mutations, accounting for about 25% of ET and about 30% of PMF patients having a CALR mutation.

MPN is a frequent form of haematological cancer. JAK2V617 mutation are highly frequent in these cancers. Current treatment regimen of these indications is administration of Ruxolitinib, Imatinib, Azacytidine, Decitabine, Hydroxyurea, and Thalidomide. However, limitations of the and low efficacy.

WO2015/068142 discloses the co-administration of chloroquine, metformin and roflumilast for the treatment of a myeloproliferative disorder. The myeloproliferative disorder comprises a mutation in Janus kinase 2, i.e. V617F, K539L and T875N, or V617F in combination with another gene mutation, amongst which KRAS, NRAS, TP53 and CDKNI A are included. Experiments run on JAK2 mutant BAF3 cells show that the addition of chloroquine to metformin decreases the viability of the cells. However said composition does not comprise a statin.

KR2006 0092428 discloses the use of simvastatin for treating chronic myelogenous leukemia. However the composition does not comprise neither chloroquine nor metformin.

In view of the limitations observed towards the treatment of MPN or its associated condition, improved or technically advanced treatment protocols for this complex disorder is needed. A safe and effective treatment and protocol that could alleviate suffering with higher efficacy and improve outcomes at lower cost would be a significant medical advance in the treatment of MPN or associated condition.

The present disclosure aims to overcome the drawbacks of the methods of the prior art and providing a combination of compounds which has higher efficacy in managing MPN.

### SUMMARY OF THE DISCLOSURE

The present disclosure relates to a combination comprising chloroquine, metformin and a statin, or a respective pharmaceutically acceptable salt or derivative thereof for use in the treatment/management of a myeloproliferative neoplasm.

Any references in the description to methods of treatment refer to the compounds, pharmaceutical compositions and medicaments of the present invention for use in a method for treatment of the human (or animal) body by therapy (or for diagnosis).

The present disclosure also relates to a combination comprising chloroquine, metformin and a statin, or a respective pharmaceutically acceptable salt or derivative thereof. In some embodiments, the combination is a single formulation/composition comprising chloroquine, metformin and a statin optionally along with pharmaceutically acceptable excipient(s). In some embodiments, the combination is a kit comprising chloroquine, metformin and statin as separate formulations optionally along with pharmaceutically acceptable excipient(s).

The present disclosure also provides use of the combinations of the present disclosure in treatment/management of myeloproliferative neoplasm and associated conditions.

The present disclosure also provides a method of treating/managing myeloproliferative neoplasm and associated conditions in a subject in need thereof, comprising administering to the subject the combinations of the present disclosure.

### BRIEF DESCRIPTION OF THE ACCOMPANYING FIGURES

In order that the disclosure may be readily understood and put into practical effect, reference will now be made to exemplary embodiments as illustrated with reference to the accompanying figures. The figures together with detailed description below, are incorporated in and form part of the specification, and serve to further illustrate the embodiments and explain various principles and advantages, in accordance with the present disclosure where:

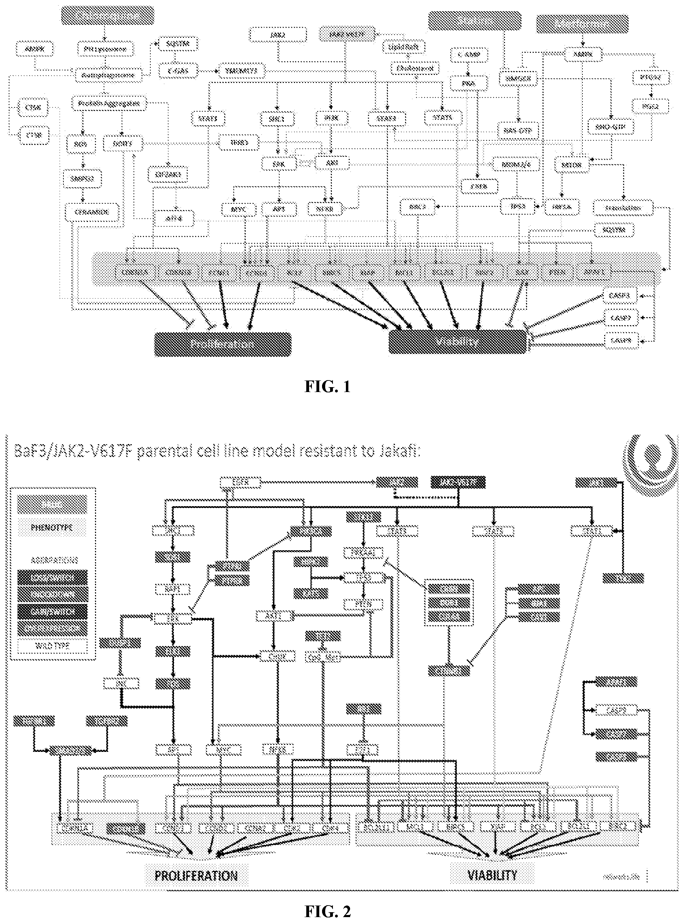

**FIG. 1** depicts the proposed mechanism of action of chloroquine + metformin + statin to reduce proliferation and viability.
**FIG. 2** depicts the signaling rationale to explain Jakafi resistance in JAK2 V617 harboring cells.

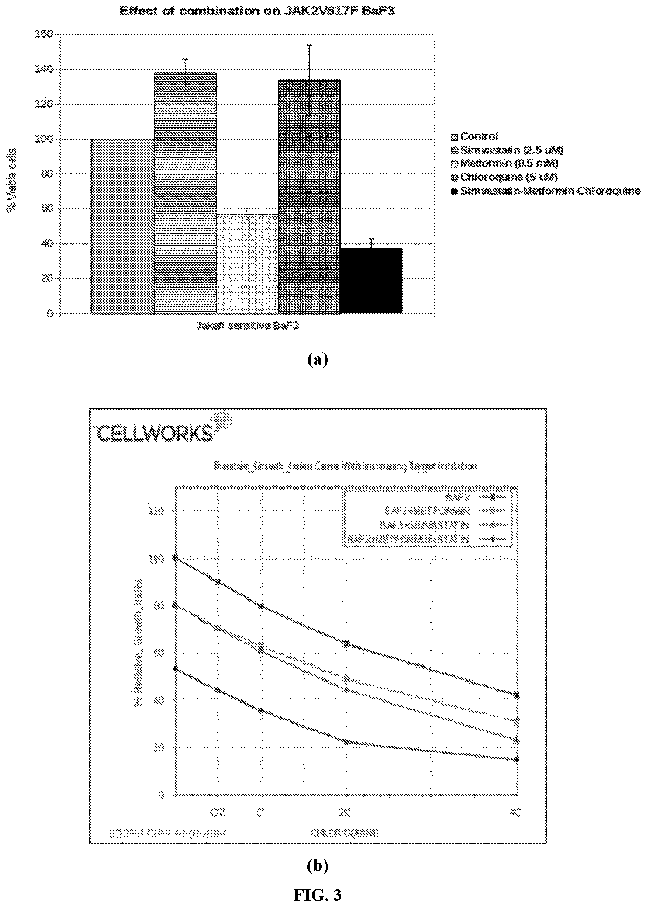

**FIG. 3****: Panel (a)** depicts experimental result of determining cell viability of BaF3 JAK2 V617F harboring cells treated with 5 µM chloroquine + 0.5 mM metformin + 2.5 µM simvastatin using MTT assay. **Panel (b)** shows the simulation result of overall relative growth on BaF3 cell avatar created using CBM and simulated with chloroquine in dose escalation manner (c/2, c, 2c, 4c swhere C is characterized with 60% inhibition of target), chloroquine + metformin, chloroquine + statin and all 3 in combinations.

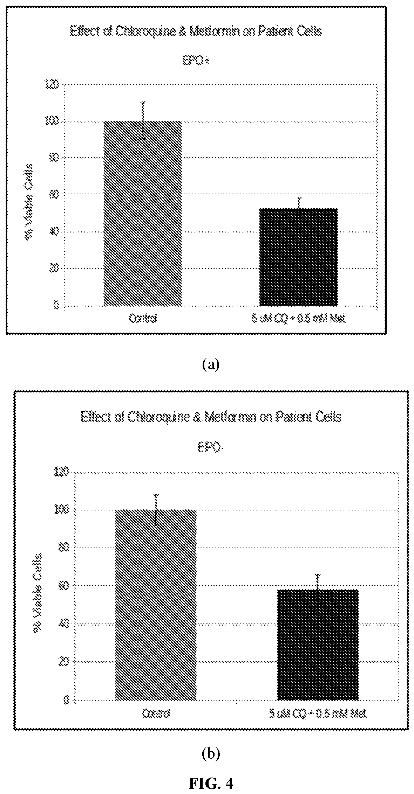

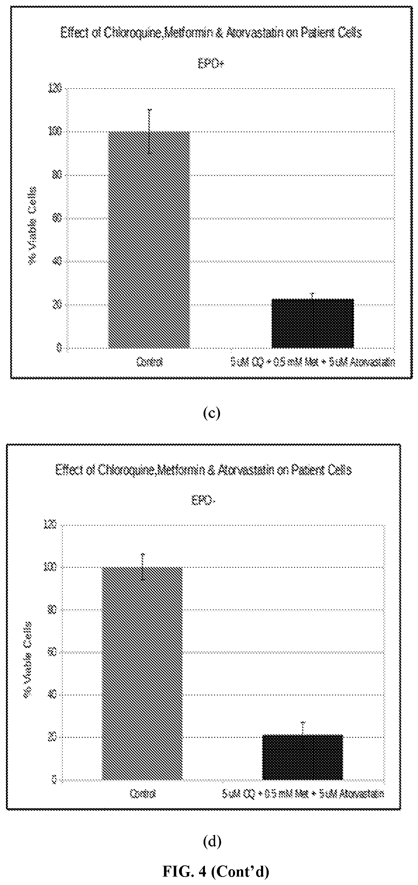

**FIG. 4****: Panel (a)** depicts results of determination of cell viability of patient cells treated with chloroquine (5 µM) and metformin (0.5 mM) in the presence of EPO [CQ + Met (EPO+)] using MTT assay. **Panel (b)** depicts the results of determination of cell viability of patient cells treated with chloroquine (5 µM) and metformin (0.5 mM) in the absence of EPO [CQ + Met (EPO-)] using MTT assay. **Panel (c)** depicts the results of determination of cell viability of patient cells treated with chloroquine (5 µM) + metformin (0.5 mM) + Atorvastatin (5 µM) in the presence of EPO [CQ + Met + Atorvastatin (EPO+)] using MTT assay. **Panel (d)** depicts the results of determination of cell viability of patient cells treated with chloroquine (5 µM) + metformin (0.5 mM) + Atorvastatin (5 µM) in the absence of EPO [CQ + Met + Atorvastatin (EPO-)] using MTT assay.

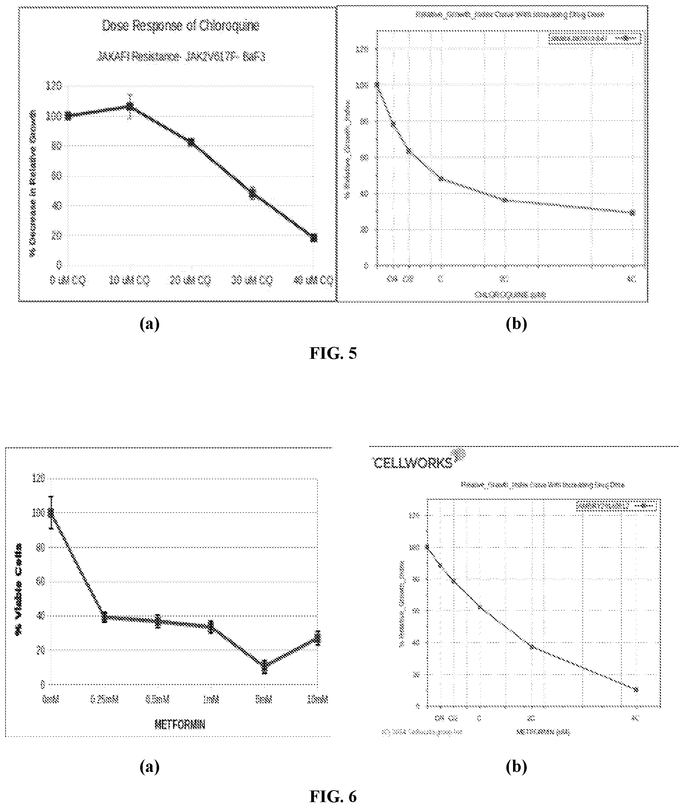

**FIG. 5****: Panel (a)** depicts the experimental results of dose escalation study performed on Jakafi resistant JAK2 V617F harboring BaF3 cells, wherein BaF3 cells were cultured with chloroquine with doses ranging from 10 µM, 20 µM, 30 µM and 40 µM, and wherein impact on relative growth was assessed. **Panel (b)** depicts the simulation result of assessing overall relative growth of Jakafi resistant JAK2 V617F BaF3 avatar created using CBM and simulated with chloroquine in dose escalation (doses of c/4, c/2, c, 2c, 4c).
**FIG. 6****: Panel (a)** depicts the experimental results of dose escalation study performed on Jakafi resistant JAK2 V617F harboring BaF3 cells, wherein BaF3 cells were cultured with metformin with doses ranging from 0.25 mM, 0.5 mM, 1 mM, 5mM and 10 mM, and impact on cell viability was assessed. **Panel (b)** depicts the simulation result of assessing overall relative growth of Jakafi resistant JAK2 V617F BaF3 avatar created using CBM and simulated with metformin in dose escalation (doses of c/4, c/2, c, 2c, 4c where C is 60% activation of target).

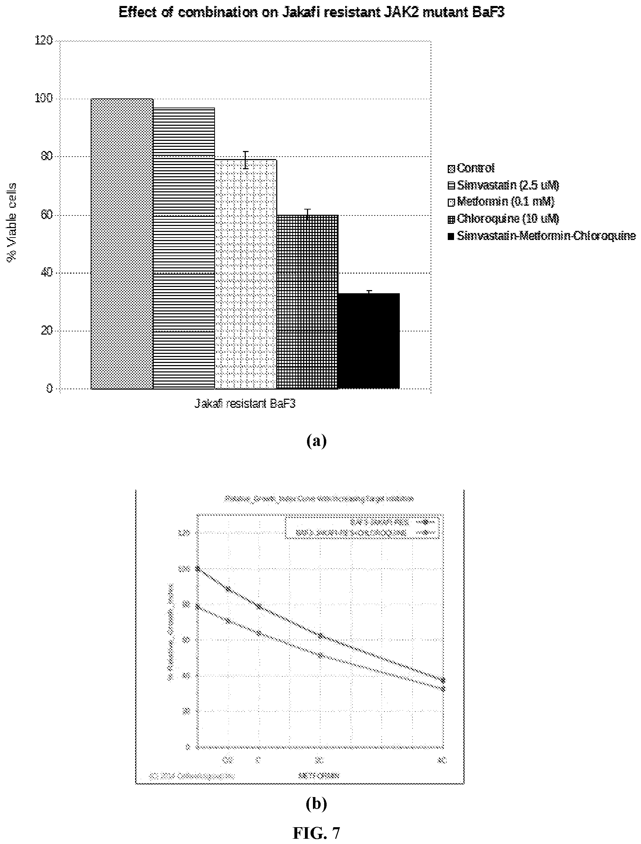

**FIG. 7****: Panel (a)** depicts the experimental results of treating Jakafi resistant JAK2 V617F harboring BaF3 cells with a combination of 10 µM chloroquine + 0.1 mM metformin + 2.5 µM simvastatin. **Panel (b)** depicts the simulation result of assessing relative growth of Jakafi resistant JAK2 V617F BaF3 cell avatar created using CBM with metformin at doses ranging from c/4, c/2, c, 2c and 4c in combination with chloroquine at fixed dose, wherein red line indicates addition of metformin at increasing doses from c/4, c/2, c, 2c and 4c, and green line indicates addition of metformin at increasing doses of c/4, c/2, c, 2c and 4c with a fixed dose of chloroquine.

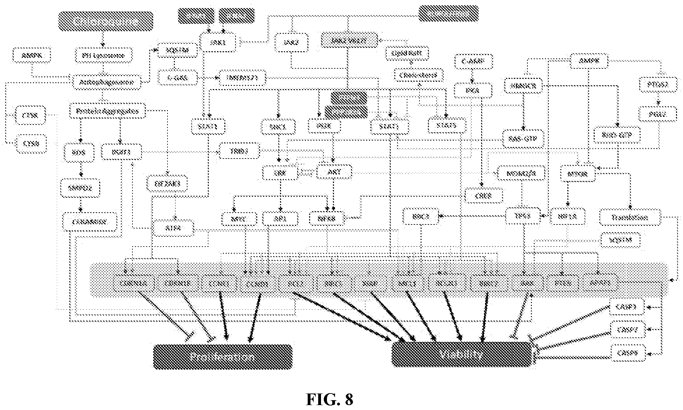

**FIG. 8** depicts the signaling rationale to explain Ruxolitinib Non-Responder cases in JAK2 aberration patient.

### DESCRIPTION OF THE DISCLOSURE

The present disclosure addresses the limitations of the art and provides a novel combination comprising chloroquine, metformin and a statin or a respective pharmaceutically acceptable salt or derivative thereof for management of myeloproliferative neoplasm, and methods thereof.

Unless otherwise defined herein, scientific and technical terms used in connection with the present disclosure shall have the meanings that are commonly understood by those of ordinary skill in the art. Further, unless otherwise required by context, singular terms shall include the plural and plural terms shall include the singular as is considered appropriate to the context and/or application. The various singular/plural permutations may be expressly set forth herein for the sake of clarity. The use of the expression "at least" or "at least one" suggests the use of one or more elements or ingredients or quantities, as the use may be in the embodiment of the disclosure to achieve one or more of the desired objects or results. Throughout this specification, the word "comprise", or variations such as "comprises" or "comprising" or "containing" or "has" or "having" wherever used, will be understood to imply the inclusion of a stated element, integer or step, or group of elements, integers or steps, but not the exclusion of any other element, integer or step, or group of elements, integers or steps.

Reference throughout this specification to "one embodiment" or "an embodiment" means that a particular feature, structure or characteristic described in connection with the embodiment may be included in at least one embodiment of the present disclosure. Thus, the appearances of the phrases "in one embodiment" or "in an embodiment" in various places throughout this specification may not necessarily all refer to the same embodiment. It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination.

The term "about" as used herein encompasses variations of +/- 10% and more preferably +/-5%, as such variations are appropriate for practicing the present invention.

As used herein, the term "cancer" employed in the context of the present disclosure refers to myeloproliferative neoplasm or associated conditions.

The terms "myeloproliferative neoplasm", "myeloproliferative disorder" and "MPN" are used interchangeably and refer to a blood cancer that begins with an abnormal mutation in a stem cell in the bone marrow.

As used herein, the expression "CQ+M+S" refers to a combination comprising chloroquine (CQ), metformin (M) and statin (S) or a respective pharmaceutically acceptable salt or derivative thereof.

As used herein, the term "combination" refers to a single composition/formulation comprising chloroquine, metformin and a statin or a respective pharmaceutically acceptable salt or derivative thereof; or a kit comprising chloroquine, metformin and a statin or a respective pharmaceutically acceptable salt or derivative thereof as separate formulations; or separate formulations/dosage forms not in the form of a kit as long as the effect achieved is commensurate with the intended purpose of the invention, i.e., to work for treatment of cancer.

Accordingly, the separate formulations comprising chloroquine, metformin and a statin or a respective pharmaceutically acceptable salt or derivative thereof may be administered simultaneously, or one after the other in any order.

As used herein, the term "combination therapy" refers to administration of chloroquine, metformin and a statin or their respective pharmaceutically acceptable salt or derivative to the subject for treatment/management of MPNs. In some embodiments, the subject receiving the combination therapy may still receive the first line of therapy. All three agents (CQ, M and S or their respective salts/derivatives) can be administered as a single formulation or separate formulations. The separate formulations can be administered concurrently. In some embodiments, concurrent administration comprises administration of three agents in any order within 10 minutes up to 3 hours of each other. In some embodiments, the combination therapy excludes administration of Roflumilast.

As used herein, the term "optional" or "optionally" means that the subsequently described event of circumstances may or may not occur, and that the description includes instances where said event or circumstance occurs and instances in which it does not. For example, "optionally treated with an additional therapeutic agent" means that in some embodiments, the subject is treated with the combination CQ+M+S and an additional therapeutic agent whereas in some other embodiments, the subject is treated with the combination CQ+M+S but not with the additional therapeutic agent.

As used herein, the term "managing" or "management" includes, treating or healing of a disease condition or disorder or ill effects or side effects. The term also encompasses maintenance of the optimum state such as regression of MPN and prevention of the further progress in the disease condition or disorder or ill effects or side effects. Further, "management" or "managing" refers to decreasing the risk of death due to a disease or disorder, delaying the onset of a disease or disorder, inhibiting the progression of a disease or disorder, partial or complete cure of a disease or disorder and/or adverse effect attributable to the said disease or disorder, obtaining a desired pharmacologic and/or physiologic effect (the effect may be prophylactic in terms of completely or partially preventing a disorder or disease or condition, or a symptom thereof and/or may be therapeutic in terms of a partial or complete cure for a disease or disorder and/or adverse effect attributable to the disease or disorder), or relieving a disease or disorder (i.e. causing regression of the disease or disorder).

As used herein, the terms "aberration", "anomaly", "mutation" and "abnormality" have the same scope and meaning and are used interchangeably. An aberration or anomaly or mutation or abnormality includes numerical, sequence as well as structural aberration.

As used herein, the term "effective amount" refers to a therapeutically effective amount or a prophylactically effective amount. A "therapeutically effective amount" refers to an amount effective, at dosages and for periods of time necessary, to achieve the desired therapeutic result, such as reduced tumor size, reduced tumor cells, increased life span or increased life expectancy. A therapeutically effective amount of a compound can vary according to factors such as the disease state, age, sex, and weight of the subject, and the ability of the compound to elicit a desired response in the subject. Dosage regimens can be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as smaller tumors, lower tumor cell numbers, increased life span, increased life expectancy or prevention of the progression of MPNs. Typically, a prophylactic dose is used in subjects prior to or at an earlier stage of disease, so that a prophylactically effective amount can be less than a therapeutically effective amount. In some embodiments, the effective amount includes the amount of active ingredient required for managing cancer, or any condition, disease or disorder arising due to cancer.

The present disclosure provides a method for treating/managing a subject having a myeloproliferative neoplasm, comprising administering to the subject a combination therapy comprising chloroquine, metformin and a statin, or a respective pharmaceutically acceptable salt or derivative thereof.

In some embodiments, the subject is suffering from a MPN selected from essential thrombocythemia, polycythemia vera, myelofibrosis, chronic myelogenous leukemia, chronic myelomonocytic leukemia, chronic neutrophilic leukemia, chronic eosionophilic leukemia, multiple myeloma, acute non lymphocytic leukemia, myelodysplasia and any combination thereof.

The term "subject" or "patient" as used herein refers to any mammal including, without limitation, humans and other primates (*e.g.,* chimpanzees and other apes and monkey species), farm animals (*e.g.,* cattle, sheep, pigs, goats and horses), domestic mammals (*e.g.,* dogs and cats), and laboratory animals (*e.g.,* rodents such as mice, rats, and guinea pigs). In some embodiments, the subject is a mammal. In some embodiments, the subject is a human. In some embodiments, the subject suffering from MPN has a mutation in JAK2, MPL and/or CALR. In some embodiments, the mutation in JAK2 is V617F, K539L, T875N, or any combination thereof.

In some embodiments, the subject suffering from MPN is responsive or sensitive to treatment with a first line of therapy (standard of care). In some embodiments, the first line of therapy comprises administration of Ruxolitinib, Azacytidine, Decitabine, Hydroxyurea, Imatinib, Thalidomide, or any combination thereof. In some embodiments, the subject is ruxolitinib-sensitive, i.e., the subject is responsive to the treatment with ruxolitinib.

In some embodiments, the subject suffering from MPN is resistant or has developed resistance to the first line of therapy. In some embodiments, the subject is ruxolitinib-resistant, i.e., the subject is not responsive to the treatment with ruxolitinib. It is possible that the subject is naturally resistant to ruxolitinib or has developed resistance to ruxolitinib due to exposure to ruxolitinib over a period of time. In some embodiments, the subject resistant to ruxolitinib comprises one or more mutations or copy number variations (CNV) in chromosome 8 and/or chromosome 9 from Tables 1 and 2.

The present disclosure is based in part on the finding that administration of a combination comprising at least three compounds viz., chloroquine, metformin and a statin, substantially reduces the number of viable tumor cells and/or proliferation of tumor cells than individual agents and aids in treating/managing myeloproliferative disorders and associated conditions with a high degree of efficacy.

### Scientific Rationale of CQ+M+S (FIG. 1)

In a JAK2 V617F harboring cell-line, disease induction and signaling is established via activation of STAT3, STATS, PI3K/AKT, mTORC1, NFkB, ERK, AP1 and increased superoxide levels. In a signaling condition with activation of said loops, the inventors hypothesized that a combination of chloroquine + metformin + a statin may prove effective in controlling increase of disease phenotypes. To explain how this combination would attain its effect (FIG.1), individual mechanism of actions of these drugs is explained below.

Metformin is an AMPK agonist known to inhibit the mTOR signaling pathway and show anti-tumor effects. It impacts cancer cell proliferation by activating AMPK which inhibits mTOR pathway, thereby reducing protein synthesis and translation of key proliferative genes. Metformin will also reduce tumor cell viability via the inhibition of mTOR/HIF/BCL2 BIRC5 axis. Metformin induced AMPK contributes to inhibiting metastasis by reduces HIF1A [via mTOR] which reduces expression of MMP2, SNAI1 and ZEB1. SNAI1 and ZEB1 induce E cadherin thus lowering cell metastasis.

Statin attains majority of its anti-tumor activity by inhibiting HMG-CoA reductase which helps in reducing tumor cell growth, proliferation, viability and metastasis. Inhibition of HMG-CoA reductase reduces prenylation of key oncogenic genes, reduction in RHEB/mTOR and RAS/ERK thus reducing cell proliferation. Statin prevents triggering of cyto-protective autophagy by reduced prenylation and activation of RAB1A. Statin's impact of reduction in Rheb GTP causes reduction in HIF1A, thus reducing anti-apoptotic genes BCL2 and BIRC5. Depleting cellular geranylgeranyl pyrophosphate causes reduction in Rhoa, Rac1, CDC42 gene activation which lowers metastasis capability of cell.

Chloroquine inhibits autophagy thereby increasing protein aggregates, resulting in multiple downstream effects leading to reduction in cell proliferation and viability. Protein aggregation leads to increased ROS levels, which via SMPD2 and ceramide increases BAX thereby inducing apoptosis. There is increased DDIT3 which via TRIB3 inhibits AKT/NFkB. Additionally, DDIT3 inhibits BCL2 reducing cell viability. Chloroquine via reduced SQSTM leads to reduction in STATS and viability related genes.

### BaF3/JAK2-V617F parental cell line model resistant to Jakafi^{®}:

Downstream pathway of JAK2-V617F includes the activation of STAT3, STATS, PI3K/AKT/NFkB and SHC1/ERK pathway. Jakafi^{®} (ruxolitinib) would ideally work in a patient with JAK2 mutation. However, the inventors surprisingly and unexpectedly found that the MPN cells may acquire Jakafi^{®} resistance by tuning down all the key pathways downstream of the driver JAK2V617F mutation. Low copy number of key genes downstream of JAK2 including STAT3, STATS, PI3K, SOS1, ELK1, FOS, and MCL1 reduced the tumor burden of JAK2V617F mutation. Low CNV of DUSP1, PTPRJ, PTPRR increases ERK; Low CNV of epigenetics regulating genes such as TET2 and KAT5; a knockdown of negative regulators of beta catenin pathway causes activation of beta catenin pathway; increase in the copy number of EGFR; low CNV of RB1 activated E2F1 transcription which in turn increases transcription of proliferative genes (Fig. 2). This leads to Jakafi^{®} resistance in JAK2 V617F BaF3 cells. Thus, there is a need to provide an alternative treatment option for treatment/management of Jakafi^{®}/ruxolitinib resistant MPNs.

In some embodiments, the combination therapy of the present disclosure provides a higher efficacy of treatment than the first line of therapy currently available for MPN. In some embodiments, the combination of the present disclosure provides a higher efficacy for treatment/management of MPN in patients resistant to Ruxolitinib (JAKAFI^{®}) but sensitive to the drug combination of CQ+M+S.

In some embodiments, at least about 10% of patients show enhanced efficacy score with CQ+M+S compared to Ruxolitinib. In some embodiments, about 10.9% of patients showed enhanced efficacy score with CQ+M+S compared to Ruxolitinib.

In some embodiments, the present disclosure is based on a finding that aberration of genes on Chromosome 8 and/or Chromosome 9 are responsible for Ruxolitinib resistance even if JAK2 activation mutation is present, and the combination of CQ+M+S is sensitive in such patient population.

The present disclosure is based in part on the finding that administration of a combination therapy comprising three agents viz., chloroquine, metformin and a statin (or their respective salt or derivative) aid in effectively treating/managing myeloproliferative disorders and associated conditions in patient population comprising Chromosome 8 aberration and/or Chromosome 9 aberration.

The statins employed in the combination therapy of the present disclosure are selected from a group comprising atorvastatin, simvastatin, rosuvastatin, fluvastatin, lovastatin, pitavastatin, pravastatin, or any combination thereof. Any of these statins are expected to work in the present combinations in view of a study conducted by Jing *et al.* where they investigated the anticancer effects of different statins and observed that almost all statins exhibited anticancer activity ("In vitro and in vivo anticancer effects of mevalonate pathway modulation on human cancer cells", Br J Cancer. 2014 Oct 14;111(8):1562-71)

In some embodiments, the combination of chloroquine, metformin and a statin or their respective salt or derivative is administered to the subject as a single formulation comprising all three agents and one or more pharmaceutically acceptable excipients.

In some embodiments, the combination of chloroquine, metformin and a statin or their respective salt or derivative is administered to the subject as separate formulations. In these embodiments, it is possible that all three agents are administered as three separate formulations, or one agent is administered as a separate formulation whereas the other two agents are in a single formulation.

In some embodiments of the methods, the single formulation or separate formulations are administered orally, parenterally and/or topically.

In some embodiments, the MPN treated according to the methods of the present disclosure comprises a mutation in one or more genes selected from a group comprising JAK2 (e.g., V617F mutation), KRAS, NRAS, TP53, CDKN1A, BRAF, EGFR, B-catenin, CDKN2A, P13KCA, APC, MYC, BCL2, SOCS1, SOCS3, TGFBR1, MCL1, TET2, ASXL1, SMAD4 or any combination thereof. In some embodiments, the cancer treated according to the methods of the present disclosure is a primary MPN. In some embodiments, the cancer treated according to the methods of the present disclosure is a metastatic MPN. In some embodiments, the cancer treated according to the methods of the present disclosure is a recurrent MPN. In some embodiments, the cancer treated according to the methods of the present disclosure is a chronic or acute MPN. In some embodiments, the cancer treated according to the methods of the present disclosure is of any grade, status, or stage, as long as a person skilled in the art or a medical practitioner deems administration of the combinations of the present disclosure fit and/or necessary for treatment of such a cancer.

Each of the compounds of the present disclosure (viz., chloroquine, metformin, statin or a pharmaceutically acceptable salt or derivative thereof and optionally the other therapeutic agent) are employed in the methods and compositions of the present disclosure in an amount that does not exceed the maximum tolerated dosage of each of the compounds individually.

In some embodiments, the present methods comprise administration of a therapeutically effective amount of the active. In some embodiments, the therapeutically effective amount of the chloroquine or a pharmaceutically acceptable salt or derivative thereof is from about 10% to about 99% of a maximum tolerated dose; the therapeutically effective amount of the metformin or a pharmaceutically acceptable salt or derivative thereof is from about 10% to about 99% of a maximum tolerated dose; and the therapeutically effective amount of the statin or a pharmaceutically acceptable salt or derivative thereof is from about 10% to about 99% of a maximum tolerated dose; and/or the therapeutically effective amount of the other therapeutic agent is from about 10% to about 99% of a maximum tolerated dose; including values and ranges therebetween.

In some embodiments, the maximum tolerated dose (MTD) of chloroquine or a pharmaceutically acceptable salt or derivative thereof is about 600 mg/day, the MTD of metformin or a pharmaceutically acceptable salt or derivative thereof is about 2500 mg/day, and the MTD of rosuvastatin or a pharmaceutically acceptable salt or derivative thereof is about 60 mg/day.

In some embodiments, the therapeutically effective amount of the chloroquine or a pharmaceutically acceptable salt or derivative thereof is ranging from about 1 mg to about 600 mg per day, preferably about 1 mg to about 500 mg, about 1 mg to about 400 mg, about 100 mg to about 300 mg, or about 200 mg to about 300 mg per day, including values and ranges therebetween.

In some embodiments, the therapeutically effective amount of the metformin or a pharmaceutically acceptable salt or derivative thereof is ranging from about 1 mg to about 2500 mg per day, preferably about 100 mg to about 2500 mg, about 200 mg to about 2500 mg, about 300 mg to about 2500 mg, about 400 mg to about 2500 mg, about 500 mg to about 2500 mg, about 600 mg to about 2500 mg, about 700 mg to about 2500 mg, about 800 mg to about 2500 mg, about 900 mg to about 2500 mg, about 1000 mg to about 2500 mg, about 1100 mg to about 2500 mg, about 1200 mg to about 2500 mg, about 1300 mg to about 2500 mg, about 1400 mg to about 2500 mg, about 1500 mg to about 2500 mg, about 1600 mg to about 2500 mg, about 1700 mg to about 2500 mg, about 1800 mg to about 2500 mg, about 1900 mg to about 2500 mg, about 2000 mg to about 2500 mg, about 2100 mg to about 2500 mg, about 2200 mg to about 2500 mg, about 2300 mg to about 2500 mg, or about 2400 mg to about 2500 mg per day, including values and ranges therebetween.

In some embodiments, the therapeutically effective amount of the statin or a pharmaceutically acceptable salt or derivative thereof is from about 1 mg to about 200 mg per day, preferably 1 mg to about 100 mg or about 50 mg to about 100 mg per day, including values and ranges therebetween.

In some embodiments of the present disclosure, the therapeutically effective amount of the chloroquine or a pharmaceutically acceptable salt or derivative thereof is ranging from about 100 mg/day to about 300 mg/day, preferably about 200 mg/day to about 300 mg/day; the therapeutically effective amount of the metformin or a pharmaceutically acceptable salt or derivative thereof is ranging from about 1500 mg/day to about 2500 mg/day, preferably about 2000 mg/day to about 2500 mg/day; and/or the therapeutically effective amount of the statin or a pharmaceutically acceptable salt or derivative thereof is ranging from about 10 mg/day to about 30 mg/day, preferably about 20 mg/day to about 30 mg/day.

In an exemplary and non-limiting embodiment of the present disclosure, the therapeutically effective amount of the chloroquine or a pharmaceutically acceptable salt or derivative thereof is about 200 mg/day; the therapeutically effective amount of the metformin or a pharmaceutically acceptable salt or derivative thereof is about 1250 mg twice a day; and/or the therapeutically effective amount of the statin (such as but not limiting to rosuvastatin) or a pharmaceutically acceptable salt or derivative thereof is about 20 mg/day.

In an exemplary embodiment, the combination drug dosage is as tabulated below:

| **Drug** | **Dosage Regime (After meal)** | | **MTD (mg/day)** | **Pharmacokinetics** | |
|---|---|---|---|---|---|
| | **Morning** | **Evening** | | **Peak Concentration at Dosage** | **t ½** |
| Chloroquine | 200 mg | | 600 | 1.18 µM | 20-60 days |
| Metformin | 1250 mg | 1250 mg | 2500 | 5.8 µM | 6.2 hours |
| Rosuvastatin | - | 20 mg | 60 | 0.09 µM | 19 hours |

In some embodiments, the foregoing values and ranges are merely suggestive. Dosages are altered depending on a number of variables, including, for example, the activity of the compound used, the disease, disorder or condition to be treated, the mode of administration, the requirements of the individual subject, the severity of the disease, disorder or condition being treated, and the judgment of the practitioner. A dose is modulated to achieve a desired pharmacokinetic or pharmacodynamics profile, such as a desired or effective blood profile.

In some embodiments, the present methods comprise administration of the formulation(s) to the subject orally, parenterally, or topically. In embodiments where chloroquine, metformin and statin are administered as separate formulations, all formulations can be administered using the same route of administration (e.g., orally, parenterally, or topically) or one formulation can be administered using one route of administration (e.g., orally, parenterally, or topically) and the other formulation(s) can be administered using the other routes of administration (e.g., if one is administered orally, the other is administered parenterally or topically or vice versa).

Parenteral administration comprises administration via injection or infusion. In some embodiments, parenteral administration is selected from intravenous, intramuscular, intradermal, subcutaneous, intratumoral, intralesional, intraperitoneal, and intrathecal administration. In some embodiments, parenteral administration is administration via intravenous infusion.

In some embodiments, BaF3 expressing Jak2-V617F cells were created using Computation Biology Modeling (CBM). Genomic aberrations of BaF3 cells were studied to identify chromosomal alterations to extract copy number changes in chromosome as well as mutations. Using this information, a predictive simulation BaF3 cells was created expressing JAK2V617F mutation. Effect of the drug agents chloroquine, metformin and statin on the BaF3 cell avatar, individually and in combinations thereof, was assessed on overall relative growth index from simulation outcome. The predictive simulation indicated synergistic efficacy of chloroquine, metformin and statin. The combination of inhibiting autophagy and prenylation machinery, and inducing AMPK using chloroquine, statin and metformin respectively, was predicted to reduce proliferation and viability of JAK2-V617F cells which have hyper-activated JAK2, STAT3, and STAT5. In some embodiments, the simulation predictions are validated by *in-vitro, ex-vivo* and *in-vivo* studies.

The administration of the combination therapy comprising chloroquine, metformin and the statin or their respective salts/derivatives is cytotoxic to MPN cancer cells, e.g., cancer cells having a JAK2 mutation and/or an aberration in chromosome 8 and/or 9. In some embodiments, the level of cytotoxicity provided by the combination therapy is statistically significantly higher than that exhibited by chloroquine, metformin and the statin alone or in a combination of any two. In some embodiments, the combination therapy comprising chloroquine, metformin and statin shows a synergistic effect against cancer cells compared to the sum of effects shown by chloroquine, metformin and statin alone.

The present disclosure contemplates administration of pharmaceutically acceptable salts or derivatives of chloroquine, metformin and a statin. Pharmaceutically acceptable salts include, for example, acid-addition salts and base-addition salts. The acid that is added to the compound to form an acid-addition salt is an organic acid or an inorganic acid. A base that is added to the compound to form a base-addition salt is an organic base or an inorganic base. In some embodiments, a pharmaceutically acceptable salt is a metal salt. In some embodiments, a pharmaceutically acceptable salt is an ammonium salt.

Metal salts arise from the addition of an inorganic base to a compound of the present disclosure. The inorganic base consists of a metal cation paired with a basic counterion, such as, for example, hydroxide, carbonate, bicarbonate, or phosphate. The metal is an alkali metal, alkaline earth metal, transition metal, or main group metal. In some embodiments, the metal is lithium, sodium, potassium, cesium, cerium, magnesium, manganese, iron, calcium, strontium, cobalt, titanium, aluminium, copper, cadmium, or zinc.

In some embodiments, a metal salt is a lithium salt, a sodium salt, a potassium salt, a cesium salt, a cerium salt, a magnesium salt, a manganese salt, an iron salt, a calcium salt, a strontium salt, a cobalt salt, a titanium salt, an aluminum salt, a copper salt, a cadmium salt, or a zinc salt.

Ammonium salts arise from the addition of ammonia or an organic amine to a compound of the present disclosure. In some embodiments, the organic amine is triethyl amine, diisopropyl amine, ethanol amine, diethanol amine, triethanol amine, morpholine, N-methylmorpholine, piperidine, N-methylpiperidine, N-ethylpiperidine, dibenzylamine, piperazine, pyridine, pyrrazole, pipyrrazole, imidazole, pyrazine, or pipyrazine.

In some embodiments, an ammonium salt is a triethyl amine salt, a diisopropyl amine salt, an ethanol amine salt, a diethanol amine salt, a triethanol amine salt, a morpholine salt, an N-methylmorpholine salt, a piperidine salt, an N-methylpiperidine salt, an N-ethylpiperidine salt, a dibenzylamine salt, a piperazine salt, a pyridine salt, a pyrrazole salt, a pipyrrazole salt, an imidazole salt, a pyrazine salt, or a pipyrazine salt.

Acid addition salts arise from the addition of an acid to a compound of the present disclosure. In some embodiments, the acid is organic. In some embodiments, the acid is inorganic. In some embodiments, the acid is hydrochloric acid, hydrobromic acid, hydroiodic acid, nitric acid, nitrous acid, sulfuric acid, sulfurous acid, a phosphoric acid, isonicotinic acid, lactic acid, salicylic acid, tartaric acid, ascorbic acid, gentisinic acid, gluconic acid, glucaronic acid, saccaric acid, formic acid, benzoic acid, glutamic acid, pantothenic acid, acetic acid, propionic acid, butyric acid, fumaric acid, succinic acid, methanesulfonic acid, ethanesulfonic acid, benzenesulfonic acid, p-toluenesulfonic acid, citric acid, oxalic acid, or maleic acid.

In some embodiments, the salt is a hydrochloride salt, a hydrobromide salt, a hydroiodide salt, a nitrate salt, a nitrite salt, a sulfate salt, a sulfite salt, a phosphate salt, isonicotinate salt, a lactate salt, a salicylate salt, a tartrate salt, an ascorbate salt, a gentisinate salt, a gluconate salt, a glucaronate salt, a saccarate salt, a formate salt, a benzoate salt, a glutamate salt, a pantothenate salt, an acetate salt, a propionate salt, a butyrate salt, a fumarate salt, a succinate salt, a methanesulfonate (mesylate) salt, an ethanesulfonate salt, a benzenesulfonate salt, a p-toluenesulfonate salt, a citrate salt, an oxalate salt, or a maleate salt.

The present disclosure provides a combination comprising chloroquine, metformin and a statin or their respective pharmaceutically acceptable salt/derivative for use in treatment of a myeloproliferative neoplasm. In some embodiments, the combination consists of chloroquine, metformin and a statin or their respective pharmaceutically acceptable salt/derivative. The MPN, the subjects in whom the combination is used, the statin, the pharmaceutically acceptable salt/derivative of chloroquine, metformin and a statin, their dosages, compositions, and routes of administration are as described herein.

The present disclosure provides use of a combination comprising chloroquine, metformin and a statin or their respective pharmaceutically acceptable salt/derivative for the preparation of a medicament for treatment of a myeloproliferative neoplasm. In some embodiments, the combination consists of chloroquine, metformin and a statin or their respective pharmaceutically acceptable salt/derivative. The MPN, the subjects in whom the combination is used, the statin, the pharmaceutically acceptable salt/derivative of chloroquine, metformin and a statin, their dosages, compositions, and routes of administration are as described herein.

The present disclosure provides a combination comprising chloroquine, metformin and a statin or their respective pharmaceutically acceptable salt/derivative. In some embodiments, the combination of chloroquine, metformin and a statin or their respective salt or derivative is a single formulation comprising all three agents and one or more pharmaceutically acceptable excipients. In some other embodiments, the combination of chloroquine, metformin and a statin or their respective salt or derivative is a kit comprising separate formulations. In these embodiments, all three agents can be provided as three separate formulations/dosage forms, or one agent can be provided as a separate formulation/dosage form while the other two agents can be in a single formulation/dosage form.

The combination of the three compounds in the present disclosure (i.e., chloroquine, metformin and a statin) reduces viability and/or proliferation of MPN cells through different mechanisms of actions.

In some embodiments, the combination of the present disclosure provides a higher efficacy in subjects suffering from MPN having a mutation in Janus Kinase 2 (JAK2). The mutations in JAK2 are as described herein.

In some embodiments, the combination of the present disclosure provides a higher efficacy in subjects sensitive/responsive to the first line of therapy for MPN compared to the efficacy provided by the first line of therapy alone.

In some embodiments, the combination of the present disclosure provides a higher efficacy in subjects sensitive/responsive to ruxolitinib compared to the efficacy provided by ruxolitinib alone.

In some embodiments, the combination of the present disclosure is effective for treating subjects resistant to the first line of therapy, e.g., administration of ruxolitinib.

In some embodiments, the combination of the present invention comprising chloroquine, metformin and a statin or their respective salt/derivative may further comprise one or more additional therapeutic agent. The additional therapeutic agent may be administered to a subject prior to, concurrently, or post administration of CQ+M+S.

In some embodiments, the combination of the present invention is administered along with the first line of therapy. That is, the patient while receiving the combination is also receiving the first line of therapy.

In some embodiments, the combination of the present disclosure comprises:
(i) chloroquine or its pharmaceutically acceptable salt/derivative, metformin or its pharmaceutically acceptable salt/derivative, and simvastatin or its pharmaceutically acceptable salt/derivative;
(ii) chloroquine or its pharmaceutically acceptable salt/derivative, metformin or its pharmaceutically acceptable salt/derivative, and atorvastatin or its pharmaceutically acceptable salt/derivative;
(iii) chloroquine or its pharmaceutically acceptable salt/derivative, metformin or its pharmaceutically acceptable salt/derivative and rosuvastatin or its pharmaceutically acceptable salt/derivative;
(iv) chloroquine or its pharmaceutically acceptable salt/derivative, metformin or its pharmaceutically acceptable salt/derivative, and Fluvastatin or its pharmaceutically acceptable salt/derivative;
(v) chloroquine or its pharmaceutically acceptable salt/derivative, metformin or its pharmaceutically acceptable salt/derivative and lovastatin or its pharmaceutically acceptable salt/derivative;
(vi) chloroquine or its pharmaceutically acceptable salt/derivative, metformin or its pharmaceutically acceptable salt/derivative, and pitavastatin or its pharmaceutically acceptable salt/derivative; or
(vii) chloroquine or its pharmaceutically acceptable salt/derivative, metformin or its pharmaceutically acceptable salt/derivative and pravastatin or its pharmaceutically acceptable salt/derivative.

In some embodiments, the combination of the present disclosure consists of:
(i) chloroquine or its pharmaceutically acceptable salt/derivative, metformin or its pharmaceutically acceptable salt/derivative, and simvastatin or its pharmaceutically acceptable salt/derivative;
(ii) chloroquine or its pharmaceutically acceptable salt/derivative, metformin or its pharmaceutically acceptable salt/derivative, and atorvastatin or its pharmaceutically acceptable salt/derivative;
(iii) chloroquine or its pharmaceutically acceptable salt/derivative, metformin or its pharmaceutically acceptable salt/derivative and rosuvastatin or its pharmaceutically acceptable salt/derivative;
(iv) chloroquine or its pharmaceutically acceptable salt/derivative, metformin or its pharmaceutically acceptable salt/derivative, and Fluvastatin or its pharmaceutically acceptable salt/derivative;
(v) chloroquine or its pharmaceutically acceptable salt/derivative, metformin or its pharmaceutically acceptable salt/derivative and lovastatin or its pharmaceutically acceptable salt/derivative;
(vi) chloroquine or its pharmaceutically acceptable salt/derivative, metformin or its pharmaceutically acceptable salt/derivative, and pitavastatin or its pharmaceutically acceptable salt/derivative; or
(vii) chloroquine or its pharmaceutically acceptable salt/derivative, metformin or its pharmaceutically acceptable salt/derivative and pravastatin or its pharmaceutically acceptable salt/derivative.

In some embodiments, chloroquine, metformin and a statin or their respective salt/derivative are present in the combination or are administered to the subject in a ratio of chloroquine: metformin: statin ranging from 0.5: 10: 1 to 8: 200: 1. In some embodiments, the ratio of chloroquine: metformin: statin is 2: 200: 1, 4: 40: 1, 1: 100: 1, 2: 40: 1, 2: 50: 1, or 5: 100: 1, and the like.

In some embodiments, chloroquine, metformin and a statin or their respective salt/derivative are present in the combination or are administered to the subject in a ratio of chloroquine: metformin: statin from 0.25: 0.5: 1 to 12: 50: 1. In some embodiments, the ratio of chloroquine: metformin: statin is 2: 4: 1, 2.5: 4: 1, 2: 5: 1, 4: 10: 1, and the like.

Each active agent of the present disclosure (viz., chloroquine, metformin, statin or a pharmaceutically acceptable salt or derivative thereof and optionally the additional therapeutic agent) is employed in the present disclosure in an amount that does not exceed its maximum tolerated dosage.

In some embodiments, each active agent described herein is present in a composition in an amount that is a fraction or percentage of the maximum tolerated amount. The maximum tolerated amount is as determined in a subject, such as a mouse or human. The fraction is expressed as a ratio of the amount present in the composition divided by the maximum tolerated dose. In some embodiments, the ratio is ranging from about 1/20 to about 1/1. In some embodiments, the ratio is about 1/20, about 1/19, about 1/18, about 1/17, about 1/16, about 1/15, about 1/14, about 1/13, about 1/12, about 1/11, about 1/10, about 1/9, about 1/8, about 1/7, about 1/6, about 1/5, about 1/4, about 1/3, about 1/2, or about 1/1. In some embodiments, the ratio is 1/20, 1/19, 1/18, 1/17, 1/16, 1/15, 1/14, 1/13, 1/12, 1/11, 1/10, 1/9, 1/8, 1/7, 1/6, 1/5, 1/4, 1/3, 1/2, or 1/1. In some embodiments, the ratio, expressed as percentage, is ranging from about 5% to about 100%.

In some embodiments, the therapeutically effective amount of the chloroquine or a pharmaceutically acceptable salt or derivative thereof is from about 10% to about 99% of a maximum tolerated dose; the therapeutically effective amount of the metformin or a pharmaceutically acceptable salt or derivative thereof is from about 10% to about 99% of a maximum tolerated dose; and the therapeutically effective amount of the statin or a pharmaceutically acceptable salt or derivative thereof is from about 10% to about 99% of a maximum tolerated dose; and/or the therapeutically effective amount of the other therapeutic agent is from about 10% to about 99% of a maximum tolerated dose; including values and ranges therebetween.

In some embodiments, the maximum tolerated dose (MTD) of chloroquine or a pharmaceutically acceptable salt or derivative thereof is about 600 mg/day, the MTD of metformin or a pharmaceutically acceptable salt or derivative thereof is about 2500 mg/day, and the MTD of rosuvastatin or a pharmaceutically acceptable salt or derivative thereof is about 60 mg/day.

In some embodiments, the therapeutically effective amount of the chloroquine or a pharmaceutically acceptable salt or derivative thereof is ranging from about 1 mg to about 600 mg per day, preferably about 1 mg to about 500 mg, about 1 mg to about 400 mg, about 100 mg to about 300 mg, or about 200 mg to about 300 mg per day, including values and ranges therebetween. Accordingly, the single formulation/dosage form or the separate formulation/dosage form is formulated to deliver the therapeutically effective amount of chloroquine or its salt/derivative as a single dose or multiple doses per day.

In some embodiments, the therapeutically effective amount of the metformin or a pharmaceutically acceptable salt or derivative thereof is ranging from about 1 mg to about 2500 mg per day, preferably about 100 mg to about 2500 mg, about 200 mg to about 2500 mg, about 300 mg to about 2500 mg, about 400 mg to about 2500 mg, about 500 mg to about 2500 mg, about 600 mg to about 2500 mg, about 700 mg to about 2500 mg, about 800 mg to about 2500 mg, about 900 mg to about 2500 mg, about 1000 mg to about 2500 mg, about 1100 mg to about 2500 mg, about 1200 mg to about 2500 mg, about 1300 mg to about 2500 mg, about 1400 mg to about 2500 mg, about 1500 mg to about 2500 mg, about 1600 mg to about 2500 mg, about 1700 mg to about 2500 mg, about 1800 mg to about 2500 mg, about 1900 mg to about 2500 mg, about 2000 mg to about 2500 mg, about 2100 mg to about 2500 mg, about 2200 mg to about 2500 mg, about 2300 mg to about 2500 mg, or about 2400 mg to about 2500 mg per day, including values and ranges therebetween. Accordingly, the single formulation/dosage form or the separate formulation/dosage form is formulated to deliver the therapeutically effective amount of metformin or its salt/derivative as a single dose or multiple doses per day.

In some embodiments, the therapeutically effective amount of the statin or a pharmaceutically acceptable salt or derivative thereof is from about 1 mg to about 200 mg per day, preferably 1 mg to about 100 mg or about 50 mg to about 100 mg per day, including values and ranges therebetween. Accordingly, the single formulation/dosage form or the separate formulation/dosage form is formulated to deliver the therapeutically effective amount of statin or its salt/derivative as a single dose or multiple doses per day.

In some embodiments, contemplated combinations/compositions of the present invention provide a therapeutically effective amount of the compounds of the present disclosure over an interval of about 30 minutes to about 8 hours after administration, enabling, for example, once-a-day, twice-a-day, three times a day, and etc. administration if desired.

In some embodiments of the present disclosure, the therapeutically effective amount of the chloroquine or a pharmaceutically acceptable salt or derivative thereof is ranging from about 100 mg/day to about 300 mg/day, preferably about 200 mg/day to about 300 mg/day; the therapeutically effective amount of the metformin or a pharmaceutically acceptable salt or derivative thereof is ranging from about 1500 mg/day to about 2500 mg/day, preferably about 2000 mg/day to about 2500 mg/day; and/or the therapeutically effective amount of the statin or a pharmaceutically acceptable salt or derivative thereof is ranging from about 10 mg/day to about 30 mg/day, preferably about 20 mg/day to about 30 mg/day.

In an exemplary and non-limiting embodiment of the present disclosure, the therapeutically effective amount of the chloroquine or a pharmaceutically acceptable salt or derivative thereof is about 200 mg/day; the therapeutically effective amount of the metformin or a pharmaceutically acceptable salt or derivative thereof is about 1250 mg twice a day; and/or the therapeutically effective amount of the statin or a pharmaceutically acceptable salt or derivative thereof is about 20 mg/day.

In an exemplary embodiment, the combination drug dosage is as tabulated below:

| **Drug** | **Dosage Regime (After meal)** | | **MTD (mg/day)** | **Pharmacokinetics** | |
|---|---|---|---|---|---|
| | **Morning** | **Evening** | | **Peak Concentration at Dosage** | **t ½** |
| Chloroquine | 200 mg | | 600 | 1.18 µM | 20-60 days |
| Metformin | 1250 mg | 1250 mg | 2500 | 5.8 µM | 6.2 hours |
| Rosuvastatin | - | 20 mg | 60 | 0.09 µM | 19 hours |

In some embodiments, the foregoing values and ranges are merely suggestive. Dosages are altered depending on a number of variables, including, for example, the activity of the compound used, the disease, disorder or condition to be treated, the mode of administration, the requirements of the individual subject, the severity of the disease, disorder or condition being treated, and the judgment of the practitioner. A dose is modulated to achieve a desired pharmacokinetic or pharmacodynamics profile, such as a desired or effective blood profile.

The combinations of the present disclosure comprising chloroquine, metformin and a statin are cytotoxic to MPN cancer cells, preferably in cancer cells having a JAK2 mutation and/or aberration in chromosome 8 and/or 9. In some embodiments, the level of cytotoxicity exhibited by the combination is statistically significantly more than that exhibited by chloroquine, metformin and statin alone or in a combination of any two. In some embodiments, the combination comprising chloroquine, metformin and statin shows a synergistic effect against cancer cells compared to the sum of effects shown by chloroquine, metformin and statin alone.

In some embodiments, the combination of the present disclosure comprising chloroquine, metformin and statin provides synergistic efficacy on the end-point markers, while dosing as low as about 10-100% of the recommended therapeutic dose of the individual agent in humans. Using a lower dose of the individual drug also provides an advantage in terms of minimizing the intensity of side-effects or toxicities associated with the drugs. Also, the drug combination works by inhibiting multiple targets minimally, so that an amplified effect is observed on all of the primary end-point markers and at the same time ensuring that all the targets have primary response ability, so as to negate the possibility of immune suppression and secondary infections. Use of smaller doses of individual drugs also lowers the cost of manufacture and formulation, providing an improved effect at lower price to the subject. Smaller doses also mitigate against wasteful administration of a drug to a physiological system that has been saturated or has reached a peak therapeutic response from smaller, synergistic doses.

In some embodiments, cell viability of tumor cells is assessed by conventional technologies, such as but not limiting to MTT assay, Clonogenic assay, etc.

In some embodiments, administration of the combinations disclosed herein comprising chloroquine, metformin and a statin or their respective salt/derivative provides about 5%, 10%, 15%, 20%, 25%, 30%, 35%, 40%, 45%, 50%, 55%, 60%, 65%, 70%, 80%, 90% or 100%, including values and ranges therebetween, reduction in the number of cancer cells compared to the number of cancer cells prior to administration of the combination.

In some embodiments, reduction in the number of cancer cells provided by the combinations disclosed herein comprising chloroquine, metformin and a statin or their respective salt/derivative is about 5%, 10%, 15%, 20%, 25%, 30%, 35%, 40%, 45%, 50%, 55%, 60%, 65%, 70%, 80%, 90% or 100% higher than that provided by chloroquine, metformin and the statin alone or in a combination of any two.

In some embodiments, administration of the combinations disclosed herein comprising chloroquine, metformin and a statin or their respective salt/derivative provides about 5%, 10%, 15%, 20%, 25%, 30%, 35%, 40%, 45%, 50%, 55%, 60%, 65%, 70%, 80%, 90% or 100%, including values and ranges therebetween, reduction in the number of JAKAFI^{®} resistant cancer cells compared to the number of cancer cells prior to administration of the combination.

In some embodiments, reduction in the number of JAKAFI^{®} resistant cancer cells provided by the combinations disclosed herein comprising chloroquine, metformin and a statin or their respective salt/derivative is about 5%, 10%, 15%, 20%, 25%, 30%, 35%, 40%, 45%, 50%, 55%, 60%, 65%, 70%, 80%, 90% or 100% higher than that provided by chloroquine, metformin and the statin alone or in a combination of any two.

In some embodiments, administration of the combinations disclosed herein comprising chloroquine, metformin and a statin or their respective salt/derivative provides about 2.5-fold, 3-fold, 4-fold, 5-fold, 7.5-fold, 8-fold, 10-fold, 12-fold, 15-fold, 20-fold, 25-fold, 30-fold, 35-fold, 40-fold, 45-fold, 50-fold, 55-fold, 60-fold, 65-fold, 70-fold, 80-fold, or 90-fold, including values and ranges therebetween, reduction in the number of cancer cells compared to the number of cancer cells prior to administration of the combination.

In some embodiments, reduction in the number of cancer cells provided by the combinations disclosed herein comprising chloroquine, metformin and a statin or their respective salt/derivative is about 2.5-fold, 3-fold, 4-fold, 5-fold, 7.5-fold, 8-fold, 10-fold, 12-fold, 15-fold, 20-fold, 25-fold, 30-fold, 35-fold, 40-fold, 45-fold, 50-fold, 55-fold, 60-fold, 65-fold, 70-fold, 80-fold, or 90-fold higher than that provided by chloroquine, metformin and the statin alone or in a combination of any two.

In some embodiments, administration of the combinations disclosed herein comprising chloroquine, metformin and a statin or their respective salt/derivative provides about 2.5-fold, 3-fold, 4-fold, 5-fold, 7.5-fold, 8-fold, 10-fold, 12-fold, 15-fold, 20-fold, 25-fold, 30-fold, 35-fold, 40-fold, 45-fold, 50-fold, 55-fold, 60-fold, 65-fold, 70-fold, 80-fold, or 90-fold, including values and ranges therebetween, reduction in the number of cancer cells compared to the number of JAKAFI^{®} resistant cancer cells prior to administration of the combination.

In some embodiments, reduction in the number of JAKAFI^{®} resistant cancer cells provided by the combinations disclosed herein comprising chloroquine, metformin and a statin or their respective salt/derivative is about 2.5-fold, 3-fold, 4-fold, 5-fold, 7.5-fold, 8-fold, 10-fold, 12-fold, 15-fold, 20-fold, 25-fold, 30-fold, 35-fold, 40-fold, 45-fold, 50-fold, 55-fold, 60-fold, 65-fold, 70-fold, 80-fold, or 90-fold higher than that provided by chloroquine, metformin and the statin alone or in a combination of any two.

The single formulation or the separate formulations comprising the chloroquine, metformin and a statin or their respective salt/derivative optionally along with additional therapeutic agents can be in any pharmaceutically acceptable dosage forms. In some embodiments, pharmaceutically acceptable dosage forms are selected from an oral dosage form, a parenteral dosage form, or a topical dosage form. Oral dosage forms can be discrete units, such as hard or soft capsules, tablets, pills, or lozenges; or a liquid form such as emulsions, solutions, suspensions, syrups, and elixirs. Parenteral dosage forms can be a liquid form such as emulsions, solutions, and suspensions or a solid form packaged in a single-dose or multidose containers that is reconstituted prior to administration. In some embodiments, parenteral dosage form is a ready-to-use (RTU) liquid form. Topical dosage forms can be solutions, collodion, suspensions, emulsions (e.g., lotions), and semisolids (e.g., foams, ointments, pastes, creams, and gels), etc.

In some embodiments, the present disclosure provides a combination comprising chloroquine, metformin and a statin or their respective salt/derivative for use in treatment/management of Myeloproliferative Neoplasm or any associated condition. In some embodiments, said combination for use in treatment/management of MPN is a single formulation comprising chloroquine, metformin and statin. In this embodiment, the other therapeutic agent(s) may be present in the same formulation as the combination of chloroquine, metformin and statin, or alternately the other therapeutic agent(s) may be administered as a separate formulation prior to, concurrently or post administration of the combination comprising chloroquine, metformin and statin. In some embodiments, said combination for use in treatment/management of MPN is a kit comprising chloroquine, metformin and statin or their respective salt/derivative optionally along with other therapeutic agents as separate formulations. In some embodiments, the present disclosure provides a combination comprising chloroquine, metformin and a statin for use in treating any myeloproliferative neoplasm or any associated condition including but not limiting to condition comprising a mutation in JAK2 preferably V617F, any cancer with JAK2 dominant profile, mutations and/or copy number variations in chromosome 8, mutations in chromosome 9, essential thrombocythemia, polycythemia vera, myelofibrosis, chronic myelogenous leukemia, chronic myelomonocytic leukemia, chronic neutrophilic leukemia, chronic eosionophilic leukemia, multiple myeloma, acute non lymphocytic leukemia, myelodysplasia or any combination of conditions thereof. The dosing amounts and the routes of administration of the combination are as disclosed herein.

In some embodiments, the present disclosure provides use of a combination comprising chloroquine, metformin and statin optionally along with other therapeutic agents in preparation of a medicament for treatment/management of Myeloproliferative Neoplasm or any associated condition. In some embodiments, said combination for use in treatment/management of MPN is a single formulation comprising chloroquine, metformin and statin. In this embodiment, the other therapeutic agent(s) may be present in the same formulation as the combination of chloroquine, metformin and statin, or alternately the other therapeutic agent(s) may be administered as a separate formulation prior to, concurrently or post administration of the combination comprising chloroquine, metformin and statin. In some embodiments, said combination for use in management of MPN is a kit comprising chloroquine, metformin and statin optionally along with other therapeutic agents as separate formulations. In some embodiments, the present disclosure provides a combination comprising chloroquine, metformin and statin for use in treating any myeloproliferative neoplasm or any associated condition including but not limiting to condition comprising mutation in JAK2 preferably V617F, any cancer with JAK2 dominant profile, mutations and/or copy number variations in chromosome 8, mutations and/or copy number variations in chromosome 9, essential thrombocythemia, polycythemia vera, myelofibrosis, chronic myelogenous leukemia, chronic neutrophilic leukemia, chronic eosionophilic leukemia, chronic myelomonocytic leukemia, multiple myeloma, acute non lymphocytic leukemia, myelodysplasia or any combination of conditions thereof, or any condition having a mutation therein. The dosing amounts and the routes of administration of the combination are as disclosed herein.

The present disclosure provides a single formulation/composition comprising chloroquine, metformin and a statin or their respective salt/derivative optionally along with one or more pharmaceutically acceptable excipient.

In addition to chloroquine, metformin and/or statin, the single formulation/composition or the multiple formulation of the present disclosure may further comprise one or more pharmaceutically acceptable excipient. Non-limiting examples of pharmaceutically acceptable excipients suitable for use in the present disclosure include carriers, stabilizers, diluents, dispersing agents, thickening agents, granulating agents, binding agents, lubricating agents, disintegrating agents, sweetening agents, glidants, anti-adherents, anti-static agents, surfactants, anti-oxidants, gums, coating agents, coloring agents, flavouring agents, coating agents, plasticizers, preservatives, suspending agents, emulsifying agents, plant cellulosic material and spheronization agents, and any combination thereof.

Non-limiting examples of pharmaceutically-acceptable excipients are found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999), each of which is incorporated by reference in its entirety.

In some embodiments, the combination / composition of the present disclosure is administered in a local or systemic manner, for example, via injection of the compound directly into an organ, optionally in a depot or sustained release formulation. Pharmaceutical compositions/formulations are provided in the form of a rapid release formulation, in the form of an extended release formulation, or in the form of an intermediate release formulation. A rapid release form provides an immediate release. An extended-release formulation provides a controlled release or a sustained delayed release.

In some embodiments, the present disclosure provides a process for preparing a composition/formulation comprising chloroquine, metformin and statin, said process comprising act of combining chloroquine, metformin and statin, optionally along with other therapeutic agent and/or pharmaceutically acceptable excipient, in any ratio, any concentration or any order thereof to obtain the composition/formulation.

The composition/formulation is modified depending upon the route of administration chosen. In some embodiments, the formulation / compositions comprising the compounds described herein are manufactured in a conventional manner, for example, by means of conventional mixing, dissolving, granulating, dragee-making, levigating, emulsifying, encapsulating, entrapping or compression processes. The compositions/formulations may include at least one pharmaceutically acceptable excipient (including carriers and diluents) and compounds described herein as free-base or pharmaceutically-acceptable salt form. The methods and pharmaceutical compositions described herein include the use of crystalline forms (also known as polymorphs), and active metabolites of these compounds having the same type of activity.

In some embodiments, methods for the preparation of compositions comprising the compounds described herein include formulating the compounds with one or more inert, pharmaceutically acceptable excipients to form a solid, semi-solid, or liquid composition. Solid compositions include, for example, powders, tablets, dispersible granules, capsules, cachets, and suppositories. Liquid compositions include, for example, solutions in which a compound is dissolved, emulsions comprising a compound, or a solution containing liposomes, micelles, or nanoparticles comprising a compound as disclosed herein. Semi-solid compositions include, for example, gels, suspensions and creams. The compositions may be in liquid solutions or suspensions, solid forms suitable for solution or suspension in a liquid prior to use, or as emulsions. These compositions may also contain minor amounts of nontoxic, auxiliary substances, such as wetting or emulsifying agents, pH buffering agents, and other pharmaceutically-acceptable additives.

The present disclosure provides a kit comprising chloroquine, metformin and statin. In some embodiments, chloroquine, metformin and statin are present as a single formulation or as separate formulations. In some embodiments, the kit comprises a single or a plurality of dosage forms. In some embodiments, the kit includes written instructions on the use of the compounds and formulations of the present disclosure. The instructions provide information on the identity of the therapeutic agent(s), modes of administration, or the indications for which the therapeutic agent(s) are used.

Pharmaceutical combinations/ compositions containing compounds described herein are administered for prophylactic and/or therapeutic treatments. In therapeutic applications, the compounds/compositions are administered to a subject already suffering from a disease or condition, in an amount sufficient to cure or at least partially arrest the symptoms of the disease or condition, or to cure, heal, improve, or ameliorate the condition itself. In prophylactic applications, the compounds/compositions are administered to a subject to avoid occurrence of disease/disorder, to increase resistance to disease/disorder, to detect and address an existing disease/disorder prior to the appearance of symptoms or to reduce negative impact of symptomatic disease.

Amounts effective for this use vary based on the severity and course of the disease or condition, previous therapy, the subject's health status, weight, and response to the drugs, and the judgment of the treating physician. Pharmaceutically acceptable amounts are determined by routine experimentation, for example, by a dose escalation clinical trial.

Multiple active agents are administered in any order or simultaneously. If simultaneously, the multiple actives are provided in a single, unified form, or in multiple forms, for example, as multiple separate pills. The compounds are packed together or separately, in a single package or in a plurality of packages. One or all of the compounds are given in multiple doses. If not simultaneous, the timing between the multiple doses may vary such as about a month.

In some embodiments, compounds of the disclosure are administered sequentially at a time interval. The time interval ranges from about 60 seconds to about 720 minutes.

In some embodiments, therapeutics is combined with genetic or genomic testing to determine whether an individual is a carrier of a mutant gene that is known to be correlated with certain diseases or conditions. A personalized medicine approach is used to provide companion diagnostic tests to discover a subject's predisposition to certain conditions and susceptibility to therapy. The companion diagnostic test is performed on a tissue sample of the subject, such as blood, hair, or skin. Instructions on the use of a companion diagnostic test are provided on written material packaged with a compound, composition, or kit of the present disclosure. The written material is, for example, a label. The written material suggests conditions or genetic features relevant to the compounds of the present disclosure. The instructions provide the subject and the supervising physician with the best guidance for achieving the optimal clinical outcome from the administration of the therapy.

In an embodiment of the present disclosure, JAK2 mutation diagnostics is used for patient selection with the inclusion criteria being JAK2 mutant positive. JAK2/V617F mutation is detected using a specific quantitative real time PCR assay in bone marrow aspirates or plasma samples.

The active agents described herein are administered before, during, or after the occurrence of a disease or condition, and the timing of administering the composition containing a compound may vary. For example, the active agents are used as a prophylactic and are administered continuously to subjects with a propensity to conditions or diseases in order to prevent the occurrence of the disease or condition. The compounds and compositions may be administered to a subject during or as soon as possible after the onset of the symptoms.

The initial administration is via any route practical, such as by any route described herein using any formulation described herein. A compound is administered as soon as is practicable after the onset of a disease or condition is detected or suspected, and for a length of time necessary for the treatment of the disease, such as, for example, from about 1 month to about 3 months. The length of treatment varies for each subject, and the length is determined using the known criteria.

In an exemplary and non-limiting embodiment, advantages of the combination, method and use of the present disclosure are selected from a group comprising but not limited to the following:
1. Synergistic efficacy: The combination of compounds provided in the present disclosure has a synergistic effect for management of MPN.
2. Higher efficacy: The combination of chloroquine, metformin and statin has higher efficacy for management of MPN compared with other first line of therapy available for MPN.
3. Higher efficacy in patient's subset where first line of therapy failed to respond: The combination of chloroquine, metformin and statin works as a responder drug where Ruxolitinib failed to respond or was responding initially and acquired resistance later.
4. Reduction of dosage and side effects: The combination reduces dosages of drugs/compounds being used in the treatment of MPN and minimizes the intensity of side-effects or toxicities associated with the drugs used in the treatment of MPN.
5. Lower cost of treatment: Cost of chloroquine, metformin and statin is much lower compared to the first line of therapy regimen available for MPN.

Additional embodiments and features of the present disclosure will be apparent to one of ordinary skill in art based on the description provided herein. The embodiments herein provide various features and advantageous details thereof in the description. Descriptions of well-known/conventional methods and techniques are omitted so as to not unnecessarily obscure the embodiments herein.

Any possible combination of two or more of the embodiments described herein is comprised within the scope of the present disclosure.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

Further, while the instant disclosure is susceptible to various modifications and alternative forms, specific aspects thereof has been shown by way of examples and drawings and are described in detail below. However, it should be understood that it is not intended to limit the invention to the particular forms disclosed, but on the contrary, the intention is to cover all modifications, equivalents, and alternatives falling within the spirit and the scope of the invention.

### EXAMPLES:

The present disclosure is further described with reference to the following examples, which are only illustrative in nature and should not be construed to limit the scope of the present disclosure in any manner.

### Example 1: In vitro Preclinical Validation- Effect of Chloroquine, Metformin and Statin in BaF3 cells expressing Jak2-V617F

To assess the impact of active agents chloroquine, metformin and a statin individually as well as in combination, BaF3 expressing Jak2-V617F cells were created using Computation Biology Modeling (CBM). Genomic aberrations of BaF3 cells were studied to identify chromosomal alterations to extract copy number changes in chromosome as well as mutations. Using this information, a predictive simulation BaF3 cells was created expressing JAK2V617F mutation. Chlorquine, metformin and statin were then simulated, individually or in combination, against the modeled BaF3/Jak2-V617F cells in the following protocol:
(i) Chloroquine was targeted on the BaF3 cell avatar in dose escalation method ranging from c/2, c, 2c, 4c and 8c,
(ii) along with dose escalation of chloroquine, metformin was added at fixed dose of [c],
(iii) along with dose escalation of chloroquine, simvastatin was added at fixed dose of [c],
(iv) along with dose escalation of chloroquine, metformin and simvastatin were added at fixed dose of [c].

MTT assay was used to assess cell viability.

Effect of drug agents on the BaF3 cell avatar was assessed on overall relative growth index from simulation outcome. Chloroquine individually was able to reduce relative growth in dose dependent manner, and the efficacy only increased when combined with metformin and statin. The predictive simulation indicated synergistic efficacy of chloroquine, metformin and statin (Fig. 3b). The combination of inhibiting autophagy and prenylation machinery, and inducing AMPK using chloroquine, statin and metformin respectively, was predicted to reduce proliferation and viability of JAK2-V617F cells which have hyper-activated JAK2, STAT3, and STAT5.

To validate the simulation predictions, BaF3/Jak2-V617F cells (University of Florida, USA) were treated with 5 µM chloroquine, 2.5 µM simvastatin and 0.5 mM metformin individually or in combination. The combination of chloroquine, simvastatin and metformin significantly reduced the viability of the cells.

### Example 2: Ex-vivo Preclinical Validation- Effect of Chloroquine, Metformin and Statin on Patient Samples

Effect of chloroquine, metformin and statin combination on human patient samples was assessed in presence or absence of erythropoietin (EPO). Erythropoietin (EPO) is a key regulator in most ex vivo protocols. It stimulates the production of red blood cells. It also acts as a major regulator of erythropoiesis by promoting the survival, proliferation, and differentiation of erythroid progenitor cells and regulating the number of erythrocytes in peripheral blood.

Tumor patient cells were cultured with chloroquine (5 µM) + metformin (0.5 mM) in the presence (FIG. 4, panel a) or absence (FIG. 4, panel b) of EPO. MTT assay was performed to asess cell viability of patient cells. A 45% reduction in viable cells was observed in precense of EPO and 41% reduction in viable cells was observed in absence of EPO, when compared to patient cells in untreated condition.

Further, patient cells were cultured with chloroquine (5 µM) + metformin (0.5mM) + Atorvastatin (5µM) in the presence (FIG. 4, panel c) or absence (FIG. 4, panel d) of EPO. MTT assay was done to asess cell viability of patient cells. A 75% reduction in viablle cells was observed in precense of EPO and 79% reduction in viable cells was observed in absence of EPO, when compared to untreated patient cells. Thus, addition of atorvastatin to chloroquine + metformin (FIG. 4, panel c) showed an added efficacy in reduction of viable patient cancer cells treated with chloroquine + metformin (FIG. 4, panel a) by 30%. Further, the results obtained illustrated that EPO did not interfere with the efficacy of the drug.

### Example 3: Effect of Chloroquine on Jakafi resistant JAK2 V617F BaF3 cell-line

The impact of chloroquine on Jakafi resistant JAK2 V617F harboring BaF3 cell-line was assessed. Chloroquine was administered in Jakafi resistant BaF3 cells (University of Florida, USA) at doses of 10 µM, 20 µM, 30 µM and 40 µM, and impact on relative growth was assessed. Relative growth of cells was assessed using MTT assay. There was a steady decrease in relative growth of BaF3 cells with increasing concentration of chloroquine (FIG. 5, panel a). Jakafi resistant JAK2 V617F BaF3 cell avatar was created using CBM and using simulations chloroquine was tested on the avatar at doses ranging from c/4, c/2, c, 2c and 4c. Relative growth was plotted and results matched as seen in *in-vitro* study with steady decrease in relative growth with increasing concentration of chloroquine (FIG. 5, panel b).

### Example 4: Effect of metformin on Jakafi resistant JAK2 V617F BaF3 cell-line

The impact of metformin on Jakafi resistant JAK2 V617F harboring BaF3 cell-line was assessed. Metformin was administered in Jakafi resistant BaF3 cells at doses of 0.25 mM, 0.5 mM, 1 mM, 5 mM and 10 mM (FIG. 6, panel a). Viable cells % was assessed using MTT assay. There was a steady decrease in cell viability of BaF3 cells with increasing concentration of metformin. Jakafi resistant JAK2 V617F BaF3 cell avatar was created using CBM and using simulations metformin was tested on avatar at doses ranging from c/4, c/2, c, 2c and 4c (FIG. 6, panel b). Cell viability was plotted and results matched as seen in *in-vitro* study with steady decrease in viability with increasing concentration of metformin.

### Example 5: Effect of chloroquine + metformin + simvastatin on Jakafi resistant JAK2 V617F BaF3 cell-line

Next, the impact of the three active agent combination - chloroquine + metformin + simvastatin - on Jakafi resistant JAK2 V617F harboring BaF3 cell-line was assessed. Jakafi resistant JAK2 V617F BaF3 cells were cultured with 10 µM chloroquine + 0.1 mM metformin + 2.5 µM simvastatin. Viable cells % was assessed using MTT assay (FIG. 7, panel a). The combination of chloroquine, simvastatin and metformin significantly reduced the viability of the cells.

Jakafi resistant JAK2 V617F BaF3 cell avatar was created using CBM and using simulations metformin was tested on avatar at doses ranging from c/4, c/2, c, 2c and 4c in combination with chloroquine at fixed dose (FIG. 7, panel b). Red line indicates addition of metformin at increasing doses from c/4, c/2, c, 2c and 4c. Relative growth was plotted which showed a reduction of 62% on relative growth. Green line indicates addition of metformin at increasing doses of c/4, c/2, c, 2c and 4c with a fixed dose of chloroquine. The efficacy of inhibiting relative growth seen was higher in combination of chloroquine + metformin than in metformin alone.

### Example 6: Efficacy comparison between MPN standard of care drugs and CQ+M+S on Cell line and patients

Efficacy comparison study of standard of care drugs in MPN Cell Lines and Patients is provided hereunder:

### Efficacy data tested on Cell Lines

- Ruxolitinib was sensitive in 75% of JAK2 Cell line populations whereas resistance in 83.33% of non JAK2 population.
- Azacytidine was sensitive in 50% of JAK2 Cell line populations whereas resistance in 50% of non JAK2 population
- Decitabine was sensitive in 50% of JAK2 Cell line populations whereas resistance in 50% of non JAK2 population
- Hydroxyurea was sensitive in 0% of JAK2 Cell line populations whereas resistance in 100% of non JAK2 population.
- Imatinib was sensitive in 0% of JAK2 Cell line populations whereas resistance in 83.33% of non JAK2 population.
- Thalidomide was sensitive in 75% of JAK2 Cell line populations whereas resistance in 16.67% of non JAK2 population.

### Efficacy of Standard of Care Drugs on patient cohort

- Ruxolitinib was sensitive in 81.18%% of JAK2 patient populations whereas resistance in 87.81% of non JAK2 population.
- Azacytidine was sensitive in 34.16% of JAK2 patients and resistance in 74% of non JAK2 patient's population.
- Decitabine was sensitive in 34.16% of JAK2 patients and resistance in 74% of non JAK2 patient's population
- Hydroxyurea was sensitive in 1 % of JAK2 patients and resistance in 100% of non JAK2 patient's population.
- Imatinib was sensitive in 0.5% of JAK2 patients and resistance in 100% of non JAK2 patient's population.
- Thalidomide was sensitive in 33.17% of JAK2 patients and resistance in 75.61% of non JAK2 patient's population.

Thus, Ruxolitinib efficacy was higher in JAK2 population compared to a drug combination of CQ+M+S.

### Example 7: Analysis of Ruxolitinib Non-Responder cases in JAK2 aberration Segment

Next, screening was done for all patient's resistant to Ruxolitinib (JAKAFI^{®}) but sensitive to the drug combination of CQ+M+S.

There were ~15 patients which showed enhanced efficacy score with CQ+M+S compared to JAKAFI and out of 15 there were 14 patient which had chromosome 9 aberration (OE) in the same region. Thus, Chromosome 9 OE is responsible for Ruxolitinib resistance.

Key findings of the present disclosure include that aberration of Chromosome 8 and Chromosome 9 is responsible for Ruxolitinib resistance even if JAK2 is aberrated and the combination of CQ+M+S is sensitive in this segment.

Further, Table 1 provides the list of genes from chromosomes 8 and 9 responsible to support the findings of the present disclosure.

**Table 1:**

| Gene | Location | Aberration | Drug | Response | Rationale |
|---|---|---|---|---|---|
| PTPN3 | Chromosome 9 | Trisomy 9 | RUXOLITINIB | Exclusion | RUXOLITINIB-IJAK2 -> STAT3/STAT5 |
| | | | | | PTPN3---ISTAT3/STATS -> BCL2L1--\|Apoptosis |
| PPP2R2A | Chromosome 8 | Trisomy 8 | RUXOLITINIB | Exclusion | RUXOLITINIB-\|JAK2 -> STAT3/STAT5 |
| | | | | | PPP2R2A---\|STAT3 -> BCL2L1--\|Apoptosis |
| IFNA1 | Chromosome 9 | Trisomy 9 | RUXOLITINIB | Exclusion | RUXOLITINIB --\| JAK2/JAK1-> STAT1 |
| | | | | | IFNA1-> IFNAR 1-> JAK1 -> STAT1-> CDKN1A/CDKN1B---\| Proliferation |
| IFNA2 | Chromosome 9 | Trisomy 9 | RUXOLITINIB | Exclusion | RUXOLITINIB --\| JAK2/JAK1-> STAT1 |
| | | | | | [FNA2-> IFNAR1-> JAK1 -> STAT1->S CDKN1A/CDKN1B---\| Proliferation |

Table 2 provides identified responder in Trisomy 8 and 9 for CQ+M+S.

**Table 2:**

| Gene | Location | Aberration | Drug | Response | Rationale |
|---|---|---|---|---|---|
| TGFBR1 | Chromosome 9 | Trisomy 9 | Metformin | Inclusion | METFORMIN->PRKAA1-SMAD2/3 |
| | | | | | TGFBR1->SMAD2/3->CANCERPROGRESSION |
| MCL1 | Chromosome 8 | Trisomy 8 | Chloroquine | Inclusion | CHLOROQUINE-\|AUTOPHAGY-\|ERSTRESS->APOPTOSIS |
| | | | | | MCL1-IBECN1->AUTOPHAGY-\|ERSTRESS |

Fig. 8 depicts the signaling rationale to explain Ruxolitinib Non-Responder cases in JAK2 aberration patient.

### Example 8: Comparison of side effects of CO + S + M and Ruxolitinib

Based on FDA and Sider database, side effects of Ruxolitinib were compared with individual Chloroquine, Rosuvastatin and Metformin. The comparison is show below.

**Table 3**

| | Jakafi/ Ruxolitinib | Rosuvastain | Metformin | Chloroquine |
|---|---|---|---|---|
| Side effects | All Grades (%) | All Grades (%) | All Grades (%) | All Grades (%) |
| Thrombocytopenia | 69.7 | Not reported | Not reported | Not reported |
| Anemia | 96.1 | Not reported | Not reported | Not reported |
| Neutropenia | 18.7 | Not reported | Not reported | Not reported |
| Headache | 14.8 | 8.5 | 5.7 | Not reported |
| Diarrhoea | Not reported | Not reported | 53 | Not reported |
| Nausea | Not reported | 6.3 | 25.5 | Not reported |

As shown in Table 3, nearly all the patients in the Jakafi/Ruxolitinib group faces Anemia and/or Thrombocytopenia. In 40-45% of patients Anemia is of grade 3 or more in Jakafi group. Many types of myeloproliferative neoplasms like myelofibrosis or essential thrombocytopenia have anemia or thrombocytopenia as their disease characteristics. In these conditions, Ruxolitinib may even worsen the outcome based on its side effects. On the contrary, grade 3/4 toxicity for chloroquine, metformin or statin is rarely reported. These drugs are well tolerated and if combined may not give high toxicity. Since efficacy of CQ + S + M combination is comparable to Jakafi; however, the side effects profile of the combination is very mild compared to ruxolitinib, this combination can be a preferred choice over Ruxolitinib (Jakafi).

## Claims

1. A combination comprising chloroquine, metformin and a statin, or a respective pharmaceutically acceptable salt for use in the treatment of a myeloproliferative neoplasm.

2. The combination for use as claimed in claim 1, wherein the myeloproliferative neoplasm is selected from essential thrombocythemia, polycythemia vera, myelofibrosis, chronic myelogenous leukemia, chronic myelomonocytic leukemia, chronic neutrophilic leukemia, chronic eosionophilic leukemia, multiple myeloma, acute non lymphocytic leukemia, myelodysplasia and any combination thereof.

3. The combination for use as claimed in claim 1 or 2, wherein the statin is selected from a group comprising simvastatin, atorvastatin, rosuvastatin, fluvastatin, lovastatin, pitavastatin, pravastatin and a combination thereof.

4. The combination for use as claimed in any one of claims 1-3, wherein the combination is used in a subject that comprises at least one mutation in Janus kinase 2 gene (JAK2) selected from V617F, K539L, T875N, or any combination thereof.

5. The combination for use as claimed in any one of claims 1-4, wherein the combination is used in a subject that comprises the mutation JAK2V617F or JAK2V617F in combination with a mutation in a gene selected from the group consisting of: KRAS, NRAS, TP53, CDKN1A, BRAF, EGFR, B-catenin, CDKN2A, P13KCA, APC, MYC, BCL2, SOCS1, SOCS3, TGFBR1, MCL1, TET2, ASXL1, SMAD4, and a combination thereof.

6. The combination for use as claimed in any one of claims 1-5, wherein the combination is used in a subject that has responded to, or is resistant to, or has developed resistance to a first line of therapy.

7. The combination for use as claimed in claim 6, wherein the first line of therapy comprises administration of Ruxolitinib, Azacytidine, Decitabine, Hydroxyurea, Imatinib, Thalidomide, or any combination thereof.

8. The combination for use as claimed in claim 6 or 7, wherein the first line of therapy comprises administration of Ruxolitinib.

9. The combination for use as claimed in any one of claims 1-8, wherein the combination is a single formulation comprising chloroquine, metformin and the statin or respective pharmaceutically acceptable salt or the combination is a kit comprising separate formulations of chloroquine, metformin and the statin or respective pharmaceutically acceptable salt.

10. The combination for use as claimed in claim 9, wherein the single formulation or separate formulations are in the form of an oral, parenteral and/or topical dosage forms.

11. The combination for use as claimed in any one of claims 1-10, wherein a ratio of chloroquine: metformin: statin or their respective salts ranges from 0.5: 10: 1 to 8: 200: 1.

12. The combination for use as claimed in any one of claims 1-10, wherein a ratio of chloroquine: metformin: statin or their respective salts ranges from 0.25: 0.5: 1 to 12: 50: 1.

## Patentansprüche

1. Kombination, umfassend Chloroquin, Metformin und ein Statin oder ein entsprechendes pharmazeutisch unbedenkliches Salz zur Verwendung bei der Behandlung eines myeloproliferativen Neoplasmas.

2. Kombination zur Verwendung nach Anspruch 1, wobei das myeloproliferative Neoplasma ausgewählt ist aus essenzieller Thrombozythämie, Polycythemia vera, Myelofibrose, chronischer myeloischer Leukämie, chronischer myelomonozytärer Leukämie, chronischer neutrophiler Leukämie, chronischer eosinophiler Leukämie, multiplem Myelom, akuter nicht lymphatischer Leukämie, Myelodysplasie und einer beliebigen Kombination davon.

3. Kombination zur Verwendung nach Anspruch 1 oder 2, wobei das Statin aus einer Gruppe ausgewählt ist, die Simvastatin, Atorvastatin, Rosuvastatin, Fluvastatin, Lovastatin, Pitavastatin, Pravastatin und eine Kombination davon umfasst.

4. Kombination zur Verwendung nach einem der Ansprüche 1-3, wobei die Kombination bei einem Individuum verwendet wird, das mindestens eine Mutation im Januskinase-2-Gen (JAK2) umfasst, ausgewählt aus V617F, K539L, T875N oder einer beliebigen Kombination davon.

5. Kombination zur Verwendung nach einem der Ansprüche 1-4, wobei die Kombination bei einem Individuum verwendet wird, das die Mutation JAK2V617F oder JAK2V617F in Kombination mit einer Mutation in einem Gen umfasst, das ausgewählt ist aus der Gruppe bestehend aus: KRAS, NRAS, TP53, CDKN1A, BRAF, EGFR, B-Catenin, CDKN2A, P13KCA, APC, MYC, BCL2, SOCS1, SOCS3, TGFBR1, MCL1, TET2, ASXL1, SMAD4 und einer Kombination davon.

6. Kombination zur Verwendung nach einem der Ansprüche 1-5, wobei die Kombination bei einem Individuum verwendet wird, das auf eine erste Therapielinie angesprochen hat oder gegen diese resistent ist oder eine Resistenz gegen diese entwickelt hat.

7. Kombination zur Verwendung nach Anspruch 6, wobei die erste Therapielinie eine Verabreichung von Ruxolitinib, Azacytidin, Decitabin, Hydroxyharnstoff, Imatinib, Thalidomid oder einer beliebigen Kombination davon umfasst.

8. Kombination zur Verwendung nach Anspruch 6 oder 7, wobei die erste Therapielinie eine Verabreichung von Ruxolitinib umfasst.

9. Kombination zur Verwendung nach einem der Ansprüche 1-8, wobei die Kombination eine einzelne Formulierung ist, die Chloroquin, Metformin und das Statin oder das jeweilige pharmazeutisch unbedenkliche Salz umfasst, oder die Kombination ein Kit ist, das separate Formulierungen von Chloroquin, Metformin und dem Statin oder dem jeweiligen pharmazeutisch unbedenklichen Salz umfasst.

10. Kombination zur Verwendung nach Anspruch 9, wobei die einzelne Formulierung oder die getrennten Formulierungen in Form einer oralen, parenteralen und/oder topischen Darreichungsform vorliegen.

11. Kombination zur Verwendung nach einem der Ansprüche 1-10, wobei ein Verhältnis von Chloroquin:Metformin:Statin oder ihren jeweiligen Salzen von 0,5:10:1 bis 8:200:1 reicht.

12. Kombination zur Verwendung nach einem der Ansprüche 1-10, wobei ein Verhältnis von Chloroquin:Metformin:Statin oder ihren jeweiligen Salzen von 0,25: 0,5:1 bis 12:50:1 reicht.

## Revendications

1. Combinaison comprenant de la chloroquine, de la metformine et de la statine, ou un sel pharmaceutiquement acceptable respectif, pour le traitement d'un néoplasme myéloprolifératif.

2. Combinaison destinée à être utilisée selon la revendication 1, dans laquelle le néoplasme myéloprolifératif est choisi parmi une thrombocythémie essentielle, une polycythémie de Vaquez, une myélofibrose, une leucémie myéloïde chronique, une leucémie myélomonocytaire chronique, une leucémie neutrophile chronique, une leucémie éosinophile chronique, un myélome multiple, une leucémie aiguë non lymphoïde, une myélodysplasie et n'importe quelle combinaison de ceux-ci.

3. Combinaison destinée à être utilisée selon la revendication 1 ou 2, dans laquelle la statine est choisie dans un groupe comprenant une simvastatine, une atorvastatine, une rosuvastatine, une fluvastatine, une lovastatine, une pitavastatine, une pravastatine et leur combinaison.

4. Combinaison destinée à être utilisée selon l'une quelconque des revendications 1 à 3, dans laquelle la combinaison est utilisée chez un sujet qui comprend au moins une mutation d'un gène Janus kinase 2 (JAK2) sélectionnée parmi V617F, K539L, T875N ou n'importe quelle combinaison de ceux-ci.

5. Combinaison destinée à être utilisée selon l'une quelconque des revendications 1 à 4, dans laquelle la combinaison est utilisée chez un sujet qui comprend la mutation JAK2V617F ou JAK2V617F en combinaison avec une mutation dans un gène sélectionné dans le groupe constitué de : KRAS, NRAS, TP53, CDKN1A, BRAF, EGFR, B-caténine, CDKN2A, P13KCA, APC, MYC, BCL2, SOCS1, SOCS3, TGFBR1, MCL1, TET2, ASXL1, SMAD4, et de leur combinaison.

6. Combinaison destinée à être utilisée selon l'une quelconque des revendications 1 à 5, dans laquelle la combinaison est utilisée chez un sujet qui a répondu, ou est résistant, ou a développé une résistance à une première ligne de thérapie.

7. Combinaison destinée à être utilisée selon la revendication 6, dans laquelle la première ligne de thérapie comprend une administration de Ruxolitinib, Azacytidine, Décitabine, Hydroxyurée, Imatinib, Thalidomide, ou de n'importe quelle combinaison de ceux-ci.

8. Combinaison destinée à être utilisée selon la revendication 6 ou 7, dans laquelle la première ligne de thérapie comprend une administration de Ruxolitinib.

9. Combinaison destinée à être utilisée selon l'une quelconque des revendications 1 à 8, dans laquelle la combinaison est une formulation unique comprenant une chloroquine, une metformine et la statine ou un sel pharmaceutiquement acceptable respectif ou la combinaison est un kit comprenant des formulations séparées de chloroquine, de metformine et de la statine ou d'un sel pharmaceutiquement acceptable respectif.

10. Combinaison destinée à être utilisée selon la revendication 9, dans laquelle la formulation unique ou les formulations séparées se présentent sous une forme pharmaceutique par voie orale, parentérale et/ou topique.

11. Combinaison destinée à être utilisée selon l'une quelconque des revendications 1 à 10, dans laquelle un rapport de chloroquine : metformine : statine ou leurs sels respectifs va de 0,5 : 10 1 à 8 : 200 : 1.

12. Combinaison destinée à être utilisée selon l'une quelconque des revendications 1 à 10, dans laquelle un rapport de chloroquine : metformine : statine ou leurs sels respectifs va de 0,25 : 0,5 : 1 à 12 : 50 : 1
